(19) Europäisches Patentamt
European Patent Office
Office européen des brevets

(11) Publication number: **0 207 370 B1**

(12) **EUROPEAN PATENT SPECIFICATION**

(45) Date of publication of patent specification: **14.04.93**

(51) Int. Cl.5: **G01N 27/40**, //C12M1/40, C12Q1/54

(21) Application number: **86108293.1**

(22) Date of filing: **18.06.86**

(54) **Electrochemical sensor and membrane therefor.**

(30) Priority: **28.06.85 US 749724**

(43) Date of publication of application:
**07.01.87 Bulletin 87/02**

(45) Publication of the grant of the patent:
**14.04.93 Bulletin 93/15**

(84) Designated Contracting States:
**AT BE CH DE FR GB IT LI NL SE**

(56) References cited:
**EP-A- 0 125 137**
**EP-A- 0 177 743**
**DE-A- 3 411 448**
**DE-B- 2 110 158**
**US-A- 3 539 455**
**US-A- 4 221 688**
**EP-A- 0 141 178**
**DE-A- 1 932 419**
**DE-B- 1 498 903**
**GB-A- 2 072 047**
**US-A- 3 819 772**
**US-A- 4 244 849**

(73) Proprietor: **MILES INC.**
**1127 Myrtle Street**
**Elkhart Indiana 46514(US)**

(72) Inventor: **Jones, James E.**
**2468 Camino Real**
**South Virginia Beach Virginia 23456(US)**

(74) Representative: **Kirchner, Dietrich, Dr. et al**
**c/o BAYER AG Konzernverwaltung RP Patentabteilung**
**W-5090 Leverkusen, Bayerwerk (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

### Background of the Invention

### A. Field of the Invention

The present invention is related to an electrochemical sensor for determining the relative concentration of glucose in a blood sample by using an enzyme catalyzed reaction, said sensor comprising an anode and a cathode which are electrically insulated from each other, a glucose permeable membrane for protecting the electrodes from contaminants and the enzyme being disposed between the membrane and the anode, the enzyme consisting of glucose oxidase, wherein the glucose oxidase is immobilized on an outer surface of said anode and the glucose permeable membrane. More particularly, the invention is directed to a new and improved whole blood, electrochemical glucose sensor including an integral, one-piece, whole blood compatible, glucose permeable membrane.

### B. Description of the Prior Art

Products that measure fluctuations in a person's blood sugar, or glucose levels have become everyday necessities for many of the nation's seven million diabetics. Because this disorder can cause dangerous anomalies in blood chemistry and is believed to be a contributor to vision loss and kidney failure, most diabetics need to test themselves periodically and adjust their glucose count accordingly, usually with insulin injections. Patients who are insulin dependent - about 10% to 15% of diabetics - are instructed by doctors to check their blood-sugar levels as often as four times daily.

For years the solution for diabetics was one of several urinanalysis kits that, despite repeated improvements, provide imprecise measurements of glucose in the blood. Examples of early urine testing for glucose are described in United States Patent Nos. 2,387,244 and 3,164,534. Later, reagent strips for urine testing were developed. Testing of urine for glucose, however, is limited in accuracy particularly since the renal threshhold for glucose spillage into the urine is different for each individual. Moreover, sugar (glucose) in urine is a sign that the glucose was too high several hours prior to the test because of the time delay in glucose reaching the urine. Readings taken from the urine, therefore, are indicative of the glucose level in the blood several hours before the urine is tested.

More accurate readings are possible by taking readings directly from blood to determine current glucose levels. The advent of home blood tests is considered by some to be the most significant advance in the care of diabetics since the discovery of insulin in 1921. Home blood glucose testing was made available with the development of reagent strips for whole blood testing. Examples of reagent strips of this type are described in United States Patent Nos. 3,164,534 and 3,092,465. A breakthrough in self-care came in 1979, when the Ames division of Miles Laboratories brought out its VISIDEX home blood testing kit. The VISIDEX home blood testing kit consists of disposable chemically coated plastic strips. When blood drawn by pricking a finger is placed on one of these strips, the resulting color change indicates the glucose content in the blood based on light reflection from the blood-contacted reagent strip.

The most significant advantages of the current technology available for home use reagent strips are low cost (roughly fifty cents per use) and a short, e.g. one minute, response time. There are significant problems with reagent strips, however. Test timing using reagent strips is very critical since exactly sixty seconds must elapse from the time a blood sample is placed on a strip to when it is removed by rinsing. The color on the strip then must be compared to a color chart. This time constraint and the necessity to visually ascertain differences in shades of colors results in extant home glucose testing kits being very user sensitive.

An alternative to reagent strips is a glucose sensor having an electrode. Electrodes are more costly and electrode technology is more complicated but the life of an electrode is weeks or months compared to the single use of a reagent strip. The response time of electrodes is very short and electrodes are not user sensitive resulting in increased accuracy over reagent strips.

There are two major types of glucose electrode sensors. The first is an electrocatalytic device which utilizes direct oxidation of glucose for obtaining a measurable response. The second is an enzyme electrode which utilizes an enzyme to convert glucose to an electroactive product which is then analyzed electrochemically.

In various electrocatalytic glucose sensors glucose is oxidized in a number of ways to form a number of intermediate or final reaction products measurable at an electrode. The reaction products can be formed chemically as well as biochemically. One chemical approach of interest is the electrochemical oxidation of

glucose. One suitable mechanism for causing the oxidation of glucose is by reaction with a suitable catalytic electrode metal in a suitable solution by application of a suitable potential.

A first step of this oxidation process is illustrated in the following equation:

$$C_6H_{12}O_6 \text{ (glucose)} \xrightarrow{\text{catalyst}} C_6H_{10}O_6 + 2H^+ + 2e^-$$

This initial step may be followed by a series of subsequent steps to produce various reaction products sensed by an electrode. The glucose can react directly at a suitable catalyst to produce a current proportional to the amount of glucose reacted. Oxygen required for the oxidation is obtained from water of solution rather than from dissolved oxygen in the sample. Alternatively, the dissipation of oxygen can be measured by an electrode, or the formation of reaction products, e.g. $H_2O_2$, can be measured to derive the concentration of glucose. Characteristics of the electrocatalytic oxidation of glucose are that no additional reagents are needed and relatively complex electronics are required to handle the circuit to give a value for glucose.

Another type of glucose sensor includes an enzyme electrode. The reactions for this electrode are given in the following equations:

$$C_6H_{12}O_6 + O_2 + H_2O \xrightarrow{\text{catalyst}} C_6H_{12}O_7 + H_2O_2$$

$$H_2O_2 \longrightarrow O_2 + 2H^+ + 2e^-$$

$$O_2 + 4H^+ \longrightarrow 2H_2O$$

In the reaction set forth in the first equation, glucose reacts with oxygen to form gluconolactone and hydrogen peroxide. This reaction is catalyzed by a suitable catalyst, such as the enzyme glucose oxidase. A suitable electrode can measure the formation of $H_2O_2$ or the decrease in oxygen, as known in the art. In either case a current is obtained which is directly related to the glucose concentration in the biological fluid assayed. For example, the oxidation of hydrogen peroxide is accomplished at a platinum anode and reduction can be accomplished at either a platinum anode or a silver cathode. In known enzyme electrodes, glucose and oxygen from diluted blood, as well as interferants such as ascorbic acid and uric acid, diffuse through a primary membrane. As glucose diffusing through this membrane reaches a secondary membrane, glucose oxidase catalyzes the conversion of the glucose to hydrogen peroxide and gluconolactone. The hydrogen peroxide may diffuse back through the primary membrane or it may further diffuse through the secondary membrane to the electrode where it can be reacted to form oxygen and a proton and produce a current proportional to the glucose concentration. Theoretically, the secondary membrane prevents passage to the electrode of substantially all molecules except hydrogen peroxide. The gluconolactone must diffuse back through the primary membrane.

Glucose electrochemical sensors are essentially made up of two major components: an electrode and a user replaceable, disposable membrane assembly including a primary membrane and a secondary membrane. The electrode is based on a Clark electrode operating in the hydrogen peroxide mode, as described in United States Patent No. 2,913,386. The Clark electrode includes a platinum anode and a silver cathode. A voltage of .7 volts is applied to the electrode and current between the cathode and anode is measured. The difference between this measured potential and that produced upon glucose reaction is proportional to the glucose concentration in the blood sample assayed.

The membrane assembly serves three distinct functions. First, the primary membrane serves to selectively allow the passage of glucose therethrough while keeping out interferants. The primary membrane, typically a plasma etched polycarbonate, is designed to have a pore size of 300 Å. An enzyme disposed between the primary and secondary membranes provides a second function of catalyzing the reaction between glucose and oxygen passing through the primary membrane to produce hydrogen peroxide. Typically, the secondary membrane is a cellulose acetate layer or can be a silicone rubber layer which is not glucose-permeable and is a relatively nonporous, extremely thin membrane which performs the third function of passing of only hydrogen peroxide therethrough. The overall function of the membrane assembly is to protect the electrode from contaminants, including glucose, while allowing hydrogen

peroxide to reach the electrode.

The electrode for detecting hydrogen peroxide is located adjacent the secondary membrane. To carry the hydrogen peroxide from the secondary membrane to the electrode, a liquid buffer is incorporated between the secondary membrane and the electrode. The electrode measures the change in concentration of the hydrogen peroxide in this buffer zone.

The primary membrane is used to separate high molecular weight blood components, e.g. proteins and cellular components of the blood, from the glucose. This membrane must be permeable to glucose but relatively impermeable to the larger molecular and cellular components of blood. The typical primary membrane is not whole blood compatible since the concentration of relatively high molecular weight components in whole blood, such as proteins, cellular components and clots foul the membrane quickly, necessitating membrane replacement.

In the reactive or enzyme layer, glucose reacts with oxygen in the presence of water and a suitable catalyst, such as glucose oxidase to produce gluconic acid (gluconolactone) and hydrogen peroxide. The reactive layer should be suitably moist to permit the enzymatic activity. The secondary membrane is a second diffusion layer where hydrogen peroxide diffuses from the reactive layer through the secondary membrane toward the electrode surface. This layer is permeable to hydrogen peroxide and water but relatively impermeable to gluconolactone and interfering substances such as ascorbic acid and uric acid. The secondary membrane has been a silicone rubber that functions as a barrier to almost all interfering low molecular weight materials, including glucose, but permits passage of hydrogen peroxide therethrough. An example of a membrane assembly with a silicone rubber secondary membrane is disclosed in United States Patent No. 3,979,274.

Silicone rubbers have not been used in the prior art for primary (glucose-permeable) membranes. Although prior art silicone rubbers are whole blood compatible and oxygen permeable, they are not glucose permeable-an essential function of a primary membrane. Prior to the present invention, no material had been found, despite the long-felt need in the art, capable of glucose and oxygen permeability while excluding the permeation of electrode interferants. A membrane material that is both oxygen and glucose permeable while preventing passage of interferants is highly desirable since with this material one membrane in the membrane assembly can be eliminated.

The typical prior art sensor also includes a layer of buffer and water adjacent the electrode surface for accumulation of hydrogen peroxide. This slows the electrode response as the hydrogen peroxide accumulates in this layer and diffuses across it before reaching the electrode. Ideally, the thickness of the buffer solution layer should be zero to provide the fastest possible electrode response. When the volume of the buffer layer is changed, which occurs during use, the electrode response range also changes. Consequently, prior art sensors require frequent recalibration of the instrument.

The buffer layer and position of the secondary membrane creates another problem. Since the reactive layer is spaced from the electrode, e.g. anode, a large percentage of the hydrogen peroxide produced at the reactive layer is washed away and never reaches the anode. This substantially reduces the sensitivity and accuracy of the electrode. It would be preferable to develop an electrode with the enzyme directly adhered to the anode. Such a construction would allow a much greater percentage of the hydrogen peroxide to reach the anode providing a highly sensitive and accurate electrode. The necessity for frequent calibration also would be reduced or eliminated.

As indicated, presently available electrochemical sensors have several disadvantages. Because the primary membrane is not whole blood compatible, only blood diluted with a buffer solution can be placed directly on the primary membrane. Also, the sensors are very expensive which limits their use to clinics. The complexity of the technology of the sensors requires use by highly skilled people further limiting use of presently available sensors to clinics rather than home use.

Before electrochemical sensors can be made usable in the home, the technology must be advanced to allow measurements using whole blood. This has not been achievable in electrochemical sensors to date because primary membranes presently used are not whole blood compatible and are quickly fouled by whole blood. The prior art electrochemical glucose sensors also permit the permeability of a substantial quantity of interferants to the buffer layer, even with measurements using diluted blood specimens, therefore decreasing measurement accuracy. These interferants are much more concentrated in whole blood so that whole blood samples cannot be measured accurately with existing electrochemical glucose sensors. Further, existing primary membrane material is not sufficiently oxygen-permeable to permit assays of whole blood, requiring blood dilution and an assay solution having an increased oxygen concentration for analysis.

In addition to whole blood, there are other body fluids from which glucose can be measured. Published data indicate that sweat is an ultrafiltrate of blood with a low, variable glucose concentration. The literature

indicates that glucose concentration in the interstitial extracellular space and intramuscular or subcutaneous locations is lower than blood glucose but this is believed to be a good measure of the blood glucose. Thus, glucose reaches the underside of the skin in potentially useful amounts.

For a sensor to function in this environment, the glucose must permeate the skin. Skin is not usually considered to be permeable to glucose; however, transcutaneous measurement of blood gases (oxygen, carbon dioxide, nitrous oxide) is well known. Since glucose is found in sweat it must permeate the skin via the sweat glands if by no other mechanism. The process of producing sweat is a dynamic process so that the glucose concentration of sweat depends on the relative permeability of the membrane of the sweat gland to glucose and water. If the process is forced to approach equilibrium, the glucose concentration should approach the concentration inside the skin. Potential practical difficulties are the metabolism of the glucose in the skin and the time required for equilibrium of glucose at the sensor.

Conventional sensors for measuring glucose in sweat require withdrawing a sample or employing elaborate procedures or testing equipment. Examples of conventional sensors are disclosed in United States Patent Nos. 4,044,772; 4,195,641; and 4,329,999. There is a need for a noninvasive sensor for measuring glucose in a body fluid such as sweat. The preferred sensor would not include elaborate equipment or require elaborate procedures.

## Summary of the Invention

An object of the present invention is to provide a new and improved whole blood electrochemical sensor for separating and measuring glucose.

Another object of the present invention is to provide a new and improved membrane permeable to glucose for determining the relative concentration of this compound present in an assay liquid.

A still further object of the present invention is to provide a new and improved electrochemical sensor that does not require special skills to set up and operate and will demonstrate accuracy and stability.

Another object of the present invention is to provide a new and improved membrane, and method of manufacturing the membrane, formed from a polymerizable silicon-containing compound dispersed in a liquid carrier removable in an amount of at least 5% by weight during polymerization in layer form.

Another object of the present invention is to provide a new and improved electrochemical sensor for quantitative analysis of glucose having a cost per use competitive with the cost of reagent strip glucose analysis.

According to the invention these objects are solved for by an electrochemical sensor including a glucose permeable membrane comprising an oxygen permeable, hydroxyl endblocked diorganopolysiloxane, preferably dimethylpolysiloxane, having an average molecular weight of at least 5,000, the membrane covering the glucose oxidase immobilized anode, thereby retaining said glucose oxidase between said membrane and said anode. The electrode of the sensor may be, for example, a Clark electrode, as disclosed in U.S. Patent 2,913,386, having a platinum anode and a silver cathode, which preferably are electrically insulated from each other by epoxy. A layer of material capable of catalyzing the reaction of glucose with oxygen, suitably an enzyme, such as glucose oxidase or glucose dehydrogenase is immobilized adjacent to, on, or integrally bonded to the electrode. The catalyst can be immobilized to the anode only or can be adhered to the entire electrode, without loss of accuracy.

The new and improved membrane formed from a dispersion of a polymerizable silicon-containing compound applied in an incompletely cured form in a liquid carrier is applied by painting or any other means over a suitable catalyst disposed near the electrode and to surround a current-sensing portion of the electrode with the membrane. The elastomer is cured in place forming a flexible elastic membrane that is oxygen and glucose-permeable and screens out most other interferants such as ascorbic acid. The unique membrane is whole blood compatible allowing the sensor to be home useable without the need for whole blood dilution. Since the membrane is whole blood compatible, oxygen and glucose-permeable and screens out most interferants, only one membrane is required in the sensor. The layer of buffer solution between the secondary membrane and the electrode required in prior art sensors can be eliminated making the sensor of the present invention easy to use, maintain and calibrate. These features make the sensor uniquely adapted for home use. The single membrane also allows bonding of the enzyme to the anode. This placement of the enzyme increases the efficiency of the sensor since hydrogen peroxide is produced directly on the current-receiving surface of the electrode. Another advantage of the membrane of the present invention is relatively high oxygen-permeability. High oxygen-permeability of the membrane provides a linear response in the relationship of glucose concentration and sensor current over the full useful range of glucose concentration. The linear response provides increased response accuracy and reliability and reduces or eliminates the need for calibration by the user. The linear response also allows

higher and lower dynamic readings than available in the prior art. Readings at these extreme levels are the most critical for diabetics.

## BRIEF DESCRIPTION OF THE DRAWINGS

The above and other objects and advantages and novel features of the present invention will become apparent from the following detailed description of the preferred embodiments of the invention illustrated in the accompanying drawings wherein:

FIG. 1 is a schematic illustration of a prior art electrochemical sensor;

FIG. 2 is a schematic illustration of the electrochemical sensor of the present invention;

FIG. 3 is a substantially vertical, cross sectional view of an electrode employed in the electrochemical sensor of the present invention;

FIG. 4 is a view taken substantially along line 4-4 of FIG. 3;

FIG. 5 is a graph illustrating sensor current versus glucose concentration using the sensor of the present invention;

FIG. 6 is a cross sectional view similar to FIG. 3 of an alternative embodiment of an electrode employed in the electrochemical sensor of the present invention.

## DETAILED DESCRIPTION OF THE PREFERRED EMBODIMENTS

Referring to the drawings, a prior art electrochemical sensor is schematically illustrated in FIG. 1 and is generally designated by the reference numeral 10. Prior art sensor 10 includes a membrane assembly consisting of a primary glucose-membrane 12 of polycarbonate that is glucose permeable; an enzyme layer 14, such as glucose oxidase, positioned behind the primary membrane 12, and a secondary membrane 16. The enzyme layer 14 catalyzes the reaction of glucose with oxygen to form reaction products of hydrogen peroxide and gluconolactone. Hydrogen peroxide passes through the hydrogen peroxide-permeable secondary membrane 16 to an electrode 18 where it is reacted to produce oxygen detected by a current received by a current sensing device in electronic readout equipment 20. Theoretically, the secondary membrane 16 protects electrode 18 from all contaminants except hydrogen peroxide. A prior art material for the secondary membrane 16 is silicone rubber which, as generally taught by the prior art, keeps out all interferring materials, including glucose. A significant disadvantage of prior art sensor 10 is that normally it is only used with diluted blood since the primary membrane 12 is not whole blood compatible.

In accordance with an important feature of the present invention an electrochemical sensor, generally designated 100, includes a whole blood-compatible, glucose-permeable membrane 112. The electrochemical sensor 100 of the present invention is schematically illustrated in FIG. 2. The membrane 112 permits whole blood to be applied directly to the membrane 112 (FIGS. 2 and 3) for measurement of glucose. The membrane 112, contrary to the teachings of the prior art, is glucose-permeable. The glucose-permeability and whole blood-compatibility of the membrane 112 eliminates the need for the secondary membrane 16 of the prior art sensor 10.

In accordance with an important feature of the present invention it has been found that a dispersion of a polymerizable silicon-containing compound applied in an incompletely cured form as a silicon compound dispersed phase in a liquid carrier, the carrier being essentially insoluble in the dispersed phase and removable from the dispersion during curing, will dry and cure as a continuous layer, film or membrane having unexpectedly high glucose-permeability to function as a single membrane in a electrochemical glucose sensor. The silicon-containing compound may be dispersed in the continuous phase as a monomer, oligomer, prepolymer, or incompletely cured polymer. The silicon compound is cured in place as a continuous polymeric coating or layer. The removable carrier removed during curing, such as by volatilization, should be included in an amount of at least 5% by weight of the dispersion, and preferably 10-90% by weight.

It has been found that the polymerizable silicon-containing compounds including monomers, oligomers, prepolymers, and incompletely cured polymers or mixtures thereof capable of polymerization or further polymerization in dispersed form will form cured layers or membranes when cured or polymerized in a dispersed layer upon removal of the continuous phase during curing to provide a layer or membrane having unexpectedly good oxygen and glucose-permeability without allowing the passage of electrode-sensitive interferants therethrough. The polymerizable silicon-containing compounds, after dispersion in a continuous phase, such as by including an emulsifier, can be cured in any known manner during removal of the continuous phase, such as by evaporation of water from a water-continuous phase silicon emulsion or dispersion, as disclosed in the Johnson et al Patent No. 4,221,688, incorporated herein by reference, or as

disclosed in Elias Patent No. 4,427,811, also incorporated herein by reference. Further, the dispersion of the silicon-containing compound can include a suitable curing catalyst or can be heat cured so long as the dispersion of the polymerizable silicon-containing compound is applied as a layer in the form of an incompletely cured dispersion and at least a portion of the carrier or continuous phase is removed from the dispersion during final curing. Without being limited to any particular mechanism, it is theorized that some alignment of the aggregating or polymerizing silicon-containing polymer molecules, during polymerization, occurs during final removal of the carrier to form micelles such that the aggregating silicon-containing polymer molecules are bound upon curing in a manner capable of permitting the permeation of glucose and oxygen between molecules while excluding electrode-sensitive interferants.

The silicon-containing compounds, useful in accordance with the invention are those which can be dispersed in an essentially insoluble liquid carrier, such as water, are polymerizable in the dispersed form, and result in a continuous film or layer upon curing.

In accordance with one embodiment of the present invention, the polymerizable silicon-containing compound is an organosiloxane, and particularly a diorganosiloxane comprising essentially a linear species of repeating diorganosiloxane units which may include small numbers of monoorganosiloxane units up to a maximum of about one monoorganosiloxane unit for each 100 diorganosiloxane units wherein the polymer chain is terminated at each end with silicone-bonded hydroxyls, as disclosed in Johnson et al. U.S. Patent No. 4,221,688, hereby incorporated by reference.

In accordance with another important embodiment of the present invention, the polymerizable silicon-containing compound forming a glucose-permeable membrane is applied onto an electrode as an aqueous silicone emulsion comprising a continuous water phase and an anionically stabilized dispersed silicone phase wherein the silicone phase is a graft copolymer of a water soluble silicate and a hydroxyl endblocked polydiorganosiloxane. As disclosed in the Saam Patent No. 4,244,849, such silicone emulsions having a pH within the range of from 8.5 to 12, are stable upon extended storage and result in a cured elastomeric continuous layer upon removal of water under ambient conditions. These silicone compounds are obtained from the interaction of hydroxyl end-blocked polydiorganosiloxanes and alkali metal silicates to form graft copolymers anionically stablized in aqueous emulsions at a pH of, for example, 8.5 to 12. If stability is not important, however, the pH is not critical. For example, the emulsion can be applied in layer form to manufacture the membrane as soon as the components are homogeneously dispersed.

The expression "hydroxyl endblocked polydiorganosiloxane" is understood to describe an essentially linear polymer of repeating diorganosiloxane units containing no more than small impurities of monoorganosiloxane units. The hydroxyl endblocked diorganosiloxane will therefore have essentially two silicon-bonded hydroxyl radicals per molecule. To impart elastomeric properties to the product obtained after removal of the water from the emulsion, the polysiloxane should have a weight average molecular weight ($M_w$) of at least 5,000. Polysiloxanes with weight average molecular weights below 5000, for example down to about 90, also are useful so long as the polymers form a continuous film or layer upon curing. Tensile strengths and elongations at break improve with increasing molecular weight with relatively high tensile strengths and elongations obtained above 50,000 $M_w$. However, since in a preferred embodiment of the invention, the cured polymers are bonded directly to an electrode and do not undergo any severe mechanical stress during use, high strength is not necessary for the polymer to be useful in the invention described herein. The maximum $M_w$ is one which can be emulsified or otherwise dispersed in a liquid carrier or continuous phase, such as water. Weight average molecular weights up to about 1,000,000 for the incompletely cured dispersed polysiloxane are expected to be practical for this invention. Upon curing, there is no upper limit to the molecular weight of the membrane. The preferred $M_w$ for the polymerizable dispersed siloxane is in the range of 1,000 to 700,000.

Organic radicals on useful hydroxyl endblocked polydiorganosiloxanes can be, for example, monovalent hydrocarbon radicals containing less than seven carbon atoms per radical and 2-(perfluoroalkyl)ethyl radicals containing less than seven carbon atoms per radical. Examples of monovalent hydrocarbon radicals include methyl, ethyl, propyl, butyl, isopropyl, pentyl, hexyl, vinyl, cyclohexyl and phenyl and examples of 2-(perfluoroalkyl)ethyl radicals include 3,3,3-trifluoropropyl and 2-(perfluorobutylmethyl). The hydroxyl endblocked polydiorganosiloxanes preferably contain organic radicals in which at least 50 percent are methyl. The preferred polydiorganosiloxanes are the hydroxyl endblocked polydimethylsiloxanes.

In accordance with one important embodiment of the present invention, the hydroxyl end-blocked polydiorganosiloxane is employed as an anionically stabilized aqueous emulsion. For the purposes of this embodiment "anionically stabilized" means the polydiorganosiloxane is stabilized in emulsion with an anionic surfactant. The most preferred anionically stabilized aqueous emulsion of hydroxyl endblocked polydiorganosiloxane are those prepared by the method of anionic emulsion polymerization described by Findlay et al. in U.S. Patent No. 3,294,725, hereby incorporated by reference to show the methods of

polymerization and to show anionically stabilized emulsions of hydroxyl endblocked polydiorganosiloxanes. Another method of preparing hydroxyl endblocked polydiorganosiloxanes is described by Hyde et al in U.S. Patent No. 2,891,920, hereby incorporated by reference to show the hydroxyl endblocked polydiorganosiloxanes and their method of preparation. These methods and others are known in the art.

An alkali metal silicate or colloidal silica can be included in the emulsified silicone composition for the preparation of extended storage stable emulsions used in the invention. The alkali metal silicates preferred for use in the emulsions forming the glucose-permeable membranes of the present invention are water soluble silicates. The alkali metal silicate is preferably employed as an aqueous solution. Aqueous silicate solutions of any of the alkali metals can be employed such as lithium silicate, sodium silicate, potassium silicate, rubidium silicate and cesium silicate.

The colloidal silicas are well known in the art and many are commercially available and can be included in the dispersion for increased strength and storage stability. Although any of the colloidal silicas can be used including fumed colloidal silicas and precipitated colloidal silicas, the preferred colloidal silicas are those which are available in an aqueous medium. Colloidal silicas in an aqueous medium are usually available in a stabilized form, such as those stabilized with sodium ion, ammonia or an aluminum ion. Aqueous colloidal silicas which have been stabilized with sodium ion are particularly useful for forming an emulsion because the pH requirement can be met by using such a sodium ion stabilized colloidal silica without having to add additional ingredients to bring the pH within the range of, for example, 8.5 to 12. The expression "colloidal silica" as used herein are those silicas which have particle diameters of from 0.0001 to 0.1 micrometers. Preferably, the particle diameters of the colloidal silicas are from 0.001 to 0.05 micrometers.

The colloidal silica can be added to the anionically stabilized hydroxylated polydiorganosiloxane in the form of a dry powder or as an aqueous dispersion. The best method is to add the colloidal silica in the form of a sodium ion stabilized aqueous dispersion of colloidal silica. There are many such sodium ion stabilized aqueous dispersions of colloidal silica which are commercially available. These commercial colloidal silicas are usually available in aqueous dispersions having from 15 to 30 weight percent colloidal silica and having a pH in the range of 8.5 to 10.5.

Aqueous solutions of sodium or potassium silicate are well known and are commercially available. The solutions generally do not contain any significant amount of discrete particles of amorphous silica and are commonly referred to as water glass. The ratio by weight of $SiO_2$ to alkali metal oxide in the aqueous solutions of alkali metal silicates is not critical and can be varied within the usual range of about 1.5 to 3.5 for the sodium silicates and 2.1 to 2.5 for the potassium silicates. The aqueous alkali metal silicate solutions are particularly useful in preparing the emulsions of the present invention because the addition of the silicate solution often brings the pH of the emulsion within the range of about 8.5 to about 12 so that additional ingredients are not necessary to adjust the pH of the emulsion. Of course, other aqueous alkali metal silicate solutions such as those prepared by hydrolyzing silicon esters in aqueous alkali metal hydroxide solutions can also be employed in the present invention.

In accordance with one embodiment of the present invention, the polymerizable silicon-containing compound is dispersed by combining an aqueous solution of an alkali metal silicate and the polymerizable silicon-containing compound in an emulsion so that a graft copolymer is formed as dispersed particles. The preferred procedure for preparing silicone emulsions is to add the alkali metal silicate to an anionically stabilized aqueous emulsion of one or more hydroxyl endblocked polydiorganosiloxanes, adjust the pH of the emulsion within the range of about 8.5 to 12 and then age the emulsion for a time period such that an elastomeric product is formed upon removal of the water under ambient conditions. In this embodiment, the pH of the emulsion containing dissolved silicate and dispersed hydroxyl end-blocked polydiorganosiloxane is important to the formation of the emulsion. A pH of 8.5 to 12 maintains the alkali metal silicate dissolved so that sufficient graft copolymerization between the dissolved silicate and dispersed siloxane occurs during removal of the carrier (e.g., water) to produce an emulsion capable of providing polymerization or further polymerization of the silicon-containing compound when deposited as a layer to form a membrane. If the pH is lower than the stated range, silicic acid is formed from the alkali metal silicate. Silicic acid is unstable and rapidly polymerizes by condensation which can gel the emulsion. Since silicic acid formation is almost completely suppressed at a pH of 10 to 12 and the reaction between dissolved alkali metal silicate and dispersed siloxanes occurs more rapidly within the pH range of 10-12, this pH range is preferred for emulsions containing an alkali metal silicate.

Silicone emulsions prepared by this silicate copolymerization embodiment are aged at a pH range of 8.5 to 12 for a time period sufficient to allow interaction between the dissolved silicate and the dispersed siloxane so that an elastomeric product is formed upon removal of the water under ambient conditions, as disclosed in Saam U.S. Pat. No. 4,244,849 hereby incorporated by reference. The aging period is effectively

reduced when an organic tin salt is employed in an amount of about 0.1 to 2 parts by weight for each 100 parts by weight of polydiorganosiloxane. The organic tin salts expected to be useful in the emulsions include mono-, di-and triorganotin salts. The anion of the tin salt employed is not critical and can be either organic or inorganic although organic anions such as carboxylates are generally preferred. Organic tin salts that can be employed include octyltin triacetate, dioctyltin dioctoate, didecyltin diacetate, dibutyltin diacetate, dibutyltin dibromide, dioctyltin dilaurate and trioctyltin acetate. The preferred diorganotin dicarboxylate is dioctyltin dilaurate.

The concentration of the polymerizable silicon-containing compound, e.g. the hydroxyl endblocked polydiorganosiloxane in the stabilized emulsion is not critical particularly since the water or other continuous phase carrier is removed during curing of the Si phase during film, layer or membrane formation.

The relative amounts of alkali metal silicates and hydroxyl endblocked polydiorganosiloxane employed can vary over a considerable range. Preferred elastomer properties are obtained when 0.3 to 30 parts by weight silicate is employed for each 100 parts by weight siloxane.

Other useful polymerizable silicon-containing compounds for forming the dispersions useful in forming a continuous silicon-containing polymer membrane having glucose-permeability in accordance with the present invention include the vinyl endblocked polydiorganosiloxanes dispersed together with an organosilicone compound having silicone-bonded hydrogen atoms, as disclosed in the Willing Patent No. 4,248,751, hereby incorporated by reference. As disclosed in the Willing patent, these silicone compounds are generally dispersed by emulsifying the vinyl endblocked polydiorganosiloxane together with an organosilicone compound having silicone-bonded hydrogen atoms using water and a surfactant to form an emulsion and thereafter adding a platinum catalyst and heating the emulsion to form a cross-linked silicone.

The vinyl endblocked polydiorganosiloxane can be any of the polydiorganosiloxanes endblocked with diorganovinylsiloxy units and can be represented by the formula

$$(CH_2=CH)R_2SiO(R_2SiO)_xSiR_2(CH=CH_2)$$

where each R is a monovalent hydrocarbon radical or a monovalent halogenated hydrocarbon radical and x is a representation of the number of repeating diorganosiloxane units in the polymer. The monovalent radicals can be any of those known in the art, but are preferably those with six carbon atoms or less. The preferred polydiorganosiloxanes are those wherein the monovalent organic radicals are methyl, ethyl, phenyl, 3,3,3-trifluoropropyl and mixtures thereof wherein at least 50 percent of the radicals are methyl radicals. The polydiorganosiloxane can be a single type polymer with the same kind of repeating diorganosiloxane units or with a combination of two or more kinds of repeating diorganosiloxane units, such as a combination of dimethylsiloxane units and methylphenylsiloxane units. A mixture of two or more polydiorganosiloxanes also is useful. The value of x is not critical since upon final curing in the dispersed layer, the value of x increases rapidly. The upper limit of polydiorganosiloxane which is suitable for this invention is limited only to the extent that it cannot be dispersed to form a homogenous dispersion to achieve a homogenous layer capable of forming a continuous membrane upon complete curing.

In accordance with this vinyl-endblocked embodiment, the organosilicone compound or mixture of compounds dispersed with the polydiorganosiloxane is one which contains silicon-bonded hydrogen atoms. The organosilicon compound can be any compound or combination of compounds containing silicon-bonded hydrogen atoms useful as crosslinkers and providing an average of silicon-bonded hydrogen atoms per molecule of organosiloxane compound of at least 2.1. Such organosilicon compounds are known in the art as illustrated in U.S. Pat. No. 3,697,473, which is hereby incorporated by reference. The preferred organosilicon compounds are those which are siloxanes made up of units selected from $HSiO_{1.5}$, $R'HSiO$, $R'_2HSiO_{0.5}$, $R'SiO_{1.5}$, $R'_2SiO$, $R'_3SiO_{0.5}$ and $SiO_2$ such that there is at least 2.1 silicon-bonded hydrogen atoms per molecule. Each R' is preferably selected from an alkyl radical of 1 to 12 carbon atoms inclusive, phenyl and 3,3,3-trifluoropropyl.

The amount of vinyl endblocked diorganosiloxane and organosilicon compound can vary broadly in weight amounts because the unit of weight for each vinyl radical or silicon-bonded hydrogen atom will vary considerably. Such "units of weight" are determined by dividing the molecular weight by the number of vinyl radicals per molecule or number of SiH per molecule. Because the cross-linked molecules in the membrane are formed by the reaction between the vinyl radical of the polydiorganosiloxane and the silicon-bonded hydrogen atom (SiH) of the organosilicon compound, the amounts of each will depend upon the ratio of SiH to vinyl. The stoichiometry would suggest that about one SiH per vinyl is all that is needed, however, the reactivity of the SiH can vary significantly, as well as its availability for reaction. For this reason, the ratio of SiH to vinyl can vary beyond the stoichiometric amounts and still provide products capable of polymerizing in layer form to provide continuous glucose-permeable membranes. The vinyl

endblocked polydiorganosiloxane and organosilicon compound preferably are combined such that the ratio of SiH to vinyl can vary from 0.75/1 to 4/1, with the most perferred range of 0.75/1 to 1.5/1.

The platinum catalyst can be any of the platinum catalysts known to catalyze the addition of silicon-bonded hydrogen atoms to silicon-bonded vinyl radicals. Platinum catalysts can be any of the known forms, ranging from platinum as such or as deposited on carriers such as silica gel or powdered charcoal, to platinic chlorides, salts of platinum and chloro-platinic acid. The dispersibilty of the platinum catalysts in the siloxane can be increased by complexing it with vinyl-containing siloxanes such as described in U.S. Pat. No. 3,419,593.

The amount of platinum catalyst used should be such that there is at least 0.1 part by weight platinum per one million parts by weight of the combined weight of polydiorganosiloxane and organosilicon compound. Preferably, the amount of catalyst used is from 1 to 20 parts by weight platinum per million parts by weight of polydiorganosiloxane and organosilicon compound. Larger amounts of platinum can be used if economic considerations are not important.

For those cases where a platinum catalyst is included in the dispersion and a platinum catalyst inhibitor is desired to prevent complete curing prior to layering the dispersion for formation of the membrane, there are many types of known inhibitors. These inhibitors retard or inhibit the activity of the platinum catalyst, but allow the platinum catalyst to become active at elevated temperatures, such as above 70° C. If the carrier in the dispersion is water, the selection of an inhibitor should be one which does not have its effectiveness destroyed by water or surfactants or it does not destroy the emulsion. Effective inhibitors include the acetylenic alcohols and other acetylenic compounds described in U.S. Pat. No. 3,445,420. Other platinum catalyst inhibitors are known as defined in U.S. Pat. No.3,188,299, U.S. Pat. No. 3,188,300, U.S. Pat. No. 3,192,181, U.S. Pat. No. 3,344,111, U.S. Pat. No. 3,383,356, U.S. Pat. No. 3,453,233, U.S. Pat. No. 3,453,234 and U.S. Pat. No. 3,532,649. The dispersed composition can be heated for a period of time to partially cross-link the Si-containing compounds to form a stable emulsion of cross-linked particles dispersed in a carrier. After application in layer form on an electrode, the layer further cures to form a continuous, glucose permeable membrane.

Evaporation of the carrier may be assisted by a flow of dry air or other gas, either at ambient temperature or at an elevated temperature, by infrared heating or a combination of the various means. Care should be taken when accelerated means are used to evaporate the carrier, e.g. water, that the rapidly leaving water vapor does not produce undesirable discontinuities in the film.

Other reinforcing materials useful for increasing the structural integrity of the cured glucose-permeable membranes of the present invention include the copolymers disclosed in the Huebner et al Patent No. 4,288,356, hereby incorporated by reference. The copolymers are emulsion polymerized and comprise free radical polymerized monomers selected from at least one unsaturated organic monomer and at least one unsaturated organosilicone monomer. The copolymers are made from 1 to 7 weight percent unsaturated organosilicon monomer and from 93 to 99 weight percent organic monomer. It is believed that any of the unsaturated organic monomers commonly used to form polymers through free radical polymerization can be used either by themselves or in combination; for example, styrene, methylmethacrylate, and vinyl chloride. The unsaturated organosilicon monomer can be an unsaturated silane, siloxane, or silazane that will copolymerize with the unsaturated organic monomer or mixture of unsaturated organic monomers used and will form SiOH under the conditions of an emulsion polymerization method used to produce the copolymer.

The unsaturated organosilicon monomer can be a silane of the formula $R'R''_xSi(R''')_{3-x}$ where R' is an olefinic unsaturated radical such as vinyl, allyl, acryloxypropyl, or methacryloxypropyl, R'' is an alkyl radical containing 1 to 4 inclusive carbon atoms or a phenyl radical, and R''' is a hydrolyzable group such as -OR'',--OCOR'', or halogen, and x is 0, 1 or 2. The unsaturated organosilicon monomer can be a cyclic siloxane of the formula $(R'R''SiO)_a$ where R' and R'' are as defined and a is from 3 to 6 inclusive. The unsaturated organosilicon monomer can be a disilazane of the formula $R'R''_2Si\text{-}NH\text{-}SiR''_2R'$ where R' and R'' are as defined. The unsaturated organosilicon monomer can be a cyclic silazane of the formula $(R'R''SiNH)_3$ where R' and R'' are as defined. A preferred unsaturated organosilicon monomer is vinyltriethoxysilane.

Examples of unsaturated organosilicon monomers include silanes such as $ViMeSiCl_2$, $ViMe_2SiOMe$, $ViMeSi(OEt)_2$, and $ViSi(OEt)_3$,siloxanes such as $(ViMe_2Si)_2O)$, $(ViMeSiO)_3$, and $(ViMeSiO)_a$ where a is 3 to 6 inclusive, and silazanes such as $(ViMe_2Si)_2NH$ and $(ViMeSiNH)_3$ where Me is methyl radical, Et is an ethyl radical and Vi is vinyl radical.

The unsaturated organic monomer and the unsaturated organosilicon monomer can be emulsion polymerized by the common methods of performing such copolymerizations. One such process is described by Blackderf in U.S. Pat. No. 3,706,697, which is hereby incorporated by reference to show a

process for copolymerizing an acrylic ester and an acryloxyalkylalkoxysilane by emulsion polymerization of the organic monomer through a free radical generator.

For example, a mixture is prepared of water and an anionic surfactant, and then a mixture of styrene and vinyltriethoxysilane is slowly added under a nitrogen blanket. Ammonium persulfate then is added as the polymerization catalyst. Heating the mixture initiates the polymerization, but it is also necessary to control the reaction temperature so that the emulsion does not overheat due to the exothermic reaction. After polymerization, the emulsion is adjusted to a pH of greater than 7.

The copolymer is added in amount of 5 to 100 parts by weight of the emulsion polymerized copolymer for each 100 parts by weight of polymerizable Si-containing compound, e.g. polydiorganosiloxane. The addition of the copolymer serves to act as a reinforcement or filler for the polydiorganosiloxane. Amounts of from 5 to 25 parts of copolymer added per 100 parts of polymerizable Si-containing compound yield a reinforced membrane having the desired glucose-permeability and strength without the addition of other fillers such as $SiO_2$. When the amount of copolymer added is from 25 to 60 parts by weight, the final product obtained by drying the emulsion is a higher strength membrane. The more copolymer added, the harder and less elastic the final membrane becomes.

In accordance with one embodiment of the invention, an alkyl tin salt is added to the dispersion to catalyze the curing of the final emulsion during the devolatilization or other removal of the carrier to yield the cured membrane. Preferred salts are dialkyltin dicarboxylates such as dibutyltindiacetate, dibutyltindilaurate, and dioctyltindilaurate. Most preferred is dibutyltindilaurate. The emulsion of catalyst is used in an amount sufficient to yield from 0.1 to 2 parts by weight of the alkyl tin salt for each 100 parts by weight of the polymerizable Si-containing compound, e.g., polydiorganosiloxane. Larger amounts could be used, but the larger amount would serve no useful purpose.

A silane cross-linking agent, of the general formula $A_m$-$Si(OR)_{4-m}$ can be added to the dispersion to enhance the physical properties of the cured membrane. The radical A, in the silane cross-linking agent is a member selected from the group consisting of a hydrogen atom, monovalent hydrocarbon radicals containing 1 to 6 inclusive carbon atoms, and monovalent halohydrocarbon radicals containing 1 to 6 inclusive carbon atoms. Preferred radicals are methyl, ethyl, phenyl, and 3,3,3-trifluoropropyl with methyl being most preferred. The radical R is a hydrogen atom, or an alkyl group containing 1 to 4 inclusive carbon atoms,

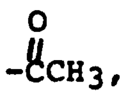,
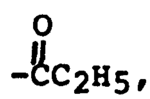,

$-CH_2CH_2OH$, $-CH_2CH_2OCH_3$, or a $-CH_2C-H_2OC_2H_5$ group. The R radicals on a silane molecule can be the same or different. The number of A radicals can be 0 or 1, meaning that a silane molecule can be either tri or tetra-functional in order to function as a cross-linker in the curing of the final membrane of this invention. The OR group on the silane is a hydrolyzable group that forms SiOH during curing of the membranes of this invention. The preferred silane cross-linking agent is methyltrimethoxysilane. The silane crosslinking agent can be included in a sufficient amount to obtain the desired degree of crosslinking. The amount to be used depends upon the hydroxyl content of the polymerizable Si-containing compound and the molecular weight of the crosslinking agent chosen. The more crosslinking agent used, the harder and less elastic the membrane becomes. Useful amounts of the preferred methyltrimethoxysilane crosslinker vary from 1 to 7 parts by weight of silane per 100 parts by weight of polydiorganosiloxane.

Other useful silicone containing compounds capable of polymerizing to form a membrane, film or layer that is glucose-permeable include the copolymers of diorganosiloxanes and any hydrolyzable silane, as disclosed in the Sorkin Patent No. 3,624,017, hereby incorporated by reference.

The diorganosiloxanes can be included in the dispersion as a monomer or a polymer. The monomer can be partially polymerized in the dispersion or emulsion and then silane added and copolymerized with the diorganosiloxane polymer. The surfactant used to form an emulsion with the copolymers can be either anionic, cationic or nonionic and any catalyst useful to initiate the copolymerization can be used, such as a strong acid or a strong base. The starting diorganosiloxane can be either a cyclic or a linear material and the molecular weight of the starting diorganosiloxane is not critical.

The dispersion of the polymerizable silicon-containing compound or compounds can contain the components in a broad range of concentrations. The preferred concentration range will depend on the thickness of the membrane desired. For example, to provide a thick elastomeric membrane (0.5 mm thick) that does not form cracks as the carrier or continuous phase evaporates, it is best to use a dispersion

having a combined amount of silicate and polydiorganosiloxane in the range of 67 to 160 parts by weight for each 100 parts by weight of carrier, e.g., water. Preferred membrane thicknesses are 0.13 to 0.64 mm (0.5 to 25 mils), for example 0.11 mm (4.5 mils).

If an emulsifying agent is incorporated into the composition to form the dispersion the amount of emulsifying agent can be less than 2 weight percent of the emulsion, and the emulsifying agent can result from neutralized sulfonic acid used in the emulsion polymerization method for the preparation of a hydroxyl endblocked polydiorganosiloxane.

Anionic surfactants are preferably the salt of the surface active sulfonic acids used in the emulsion polymerization to form the hydroxyl endblocked polydiorganosiloxane as shown in U.S. Pat. No. 3,294,725, hereby incorporated by reference to show the surface active sulfonic acids and salts thereof. The alkali metal salts of the sulfonic acids are preferred, particularly the sodium salts. The sulfonic acid can be illustrated by aliphatically substituted benzenesulfonic acids, aliphatically substituted naphthalene sulfonic acids, aliphatic sulfonic acids, silylalkylsulfonic acids and aliphatically substituted diphenylethersulfonic acids. Other anionic emulsifying agents can be used, for example, alkali metal sulforicinoleates, sulfonated glyceryl esters of fatty acids, salts of sulfonated monovalent alcohol esters, amides of amino sulfonic acid such as the sodium salt of oleyl methyltauride, sulfonated aromatic hydrocarbon alkali salts such as sodium alpha-naphthalene monosulfonate, condensation products of naphthalene sulfonic acids with formaldehyde, and sulfates such as ammonium lauryl sulfate, triethanol amine lauryl sulfate and sodium lauryl ether sulfate.

Nonionic emulsifying agents also can be included in the emulsion in addition to the anionic emulsifying agents. Such nonionic emulsifying agents are, for example, saponins, condensation products of fatty acids with ethylene oxide such as dodecyl ether of tetraethylene oxide, condensation products of ethylene oxide and sorbitan trioleate, condensation products of phenolic compounds having side chains with ethylene oxide such as condensation products of ethylene oxide with isododecylphenol, and imine derivatives such as polymerized ethylene imine.

The polymerizable silicon-compound dispersion used to form the glucose-permeable membranes of the present invention may contain additional ingredients to modify the properties of the dispersions or the cured polymeric membrane products obtained from the dispersion. For example, a thickener may be added to modify viscosity of the dispersion or to provide thixotropy for the dispersion. An antifoam agent may be added to the dispersion to reduce foaming during preparation, coating or curing in layer form.

Fillers may be added to the dispersion to reinforce, extend or pigment the membrane. Useful fillers include colloidal silica, carbon black, clay, alumina, calcium carbonate, quartz, zinc oxide, mica, titanium dioxide and others well known in the art. These fillers should be finely divided and it may be advantageous to use aqueous dispersions of such fillers if they are commercially available, such as aqueous dispersions of carbon black. The polymerizable Si-compound containing dispersions do not require a filler and such can be added in dry or aqueous forms to provide selected properties to the membrane.

The filler preferably has an average particle diameter of less than 10 micrometers. Useful fillers have had average particle diameters ranging down to as low as 0.05 micrometers. When these silicone emulsions are spread out for final curing to form the glucose-permeable membranes of the present invention, the water or other nonsolvent carrier evaporates, or is otherwise removed, to leave a cured glucose and oxygen-permeable membrane. Evaporation of the carrier is usually complete within a few hours to about one day depending on the dispersion film thickness and method of application. Another of the important advantages of the present invention is the excellent adhesion shown by these membranes for both polar and nonpolar substrates.

It should be understood that this invention is not limited to removal of continuous liquid phase in the silicon dispersion by evaporation, since other methods such as coagulation may be useful. Heating the polymerizable silicon-containing dispersions to more rapidly remove the carrier to produce more rapidly cured membranes also may be advantageous.

In accordance with the present invention, the glucose-permeable membranes 112 disclosed herein are useful in conjunction with any known method and apparatus for measuring the concentration of glucose permeating the membrane. More specifically, glucose concentrations have been determined amperometrically using soluble glucose oxidase held between Cupropane membranes or physically entrapped in a polyacrylamide gel coated onto an oxygen electrode. The decrease in oxygen pressure is equivalent to the glucose content in the biological fluid, such as blood or plasma, in accordance with the reaction:

$$\text{glucose}+O_2+H_2O \xrightarrow{\text{glucose oxidase}} H_2O_2+\text{gluconic acid.}$$

Instead of measuring the decrease in oxygen content, the hydrogen peroxide produced in the enzymatic reaction is an alternative use for the glucose-permeable membrane. Such a device for measuring the hydrogen peroxide is disclosed in the Clark U.S. Patent No. 3,539,455.

Some presently existing apparatus uses glucose oxidase held on a filter trap and utilizes two platinum electrodes, one to compensate for any electrooxidizable compounds in the sample, such as ascorbic acid, and the second to monitor the enzyme reaction producing the hydrogen peroxide. Others also have used quinone as the hydrogen accepter in place of oxygen and measure the electro oxidation of quinone in accordance with the reaction:

$$\text{glucose}+\text{quinone}+H_2O \xrightarrow{\text{Glucose oxidase}} \text{gluconic acid + hydroquinone}$$

$$\text{hydroquinone} \xrightarrow{\text{Pt}} \text{quinone} + 2H^+\ 2e^-$$

(E = 0.4 V vs. standard calomel electrode) In such a quinone electrooxidation, glucose oxidase is trapped in a porous gelled layer and covered with a dialysis membrane over a platinum electrode. Others have immobilized glucose oxidase onto a platinum-glass electrode held in place by cellophane. The current produced is proportional to the glucose concentration. Others have measured the local decrease in iodide activity at an electrode surface either in a flow stream or at a stationary electrode in accordance with the following catalyzed reactions:

$$\text{glucose} + O_2 \xrightarrow{\text{glucose oxidase}} \text{gluconic acid} + H_2O^2$$

$$H_2O_2 + 2I^- + 2H^+ \xrightarrow{\text{peroxidase}} 2H_2O + I_2$$

While, in the prior art, such an electrode measurement of glucose required removal of interfering reducing agents, such as ascorbic acid, the glucose-permeable membranes of the present invention are very selective to permeation of glucose and oxygen while preventing the permeation of electrode sensitive reducing agents. Accordingly, the membranes of the present invention are also very well suited to such electrode measurement systems.

One of the more important advantages to the glucose-permeable membranes of the present invention, however, is the capability of these membranes to be bonded to an electrode activated with a bonded layer of a suitable catalyst, such as glucose oxidase, or glucose dehydrogenase to eliminate any necessity for any intermediate layer of ion-conducting buffer solution. In accordance with this embodiment of the present invention a compound capable of catalyzing the reaction of glucose with oxygen is bonded directly to the electrode 118, e.g., the anode 122, and the glucose-permeable membrane 112 of the present invention is coated over the catalyzed anode 122 to entrap the catalyst 114 between the membrane layer 112 and the anode 122 outer surface. The catalyst, e.g., glucose oxidase 114, is immobilized on the outer surface of the electrode 118 in any manner.

In accordance with an important embodiment of the present invention, the electrochemical sensor 100 of the present invention is manufactured without a buffer solution adjacent the electrode 118 by immobilizing the catalyst, e.g., glucose oxidase enzyme 114, directly onto an outer surface of the electrode 118. Electrode 118, for example, can be a Clark electrode with a platinum anode 122 (FIGS. 3 and 4) surrounded by an electrically insulating epoxy ring 124 and silver cathode 126 surrounding the epoxy ring 124. Anode

122, ring 124 and cathode 126 define a working surface 127. For example, a potential of 0.7 volt is applied across anode 122 and cathode 126 and the current is measured for calibration. Catalyst 114 is bonded directly to anode 122 to maximize the efficiency, and accuracy of the sensor 100, and to minimize response time. Direct catalyst coating onto electrode 118 minimizes the distance a peroxide molecule must travel to the electrode surface after it has been formed by reaction. By direct coating of catalyst 114 onto the electrode 118, the buffer layer can be eliminated between the prior art secondary membrane 16 and electrode 18. These features reduce the distance the peroxide molecules must travel to the electrode surface reducing the time before the hydrogen peroxide is received and sensed by electrode 118. Also, the concentration of hydrogen peroxide at electrode 118 is higher which contributes to the improved linear response, efficiency, accuracy and response time of sensor 100.

Immobilization of enzyme 114 directly on anode 122 can be accomplished in several ways known in the art. For example, immobilization can be accomplished through a silane coupling agent such as N-beta-aminoethyl-gamma-aminopropyltrimethoxy silane. An important characteristic of silane coupling agents is their ability to form covalent bonds with many metal oxides and hydroxylated metal surfaces at the $Si(OR_3)$ portion of the silane molecule. This is true with platinum. Upon normal exposure to ambient conditions, platinum readily develops an hydroxy functional surface. An alkoxysilane rapidly reacts with this surface to form stable "Pt-O-Si" bonds. The silane coupling agents also include an organofunctional, e.g., amino, group that reacts with the catalyst, such as glucose oxidase 114, via a suitable crosslinking agent, such as glutaraldehyde, to immobilize the oxidizing enzyme, e.g., glucose oxidase 114, directly onto the anode pin 122 and the bonded enzyme 114 remains active for several months.

To achieve the full advantage of the present invention, the catalyst 114 capable of catalyzing the reaction of glucose with oxygen is immobilized on the surface of the anode 122 using a silane coupling agent and a suitable crosslinking agent. Crosslinking agents suitable for immobilizing a protein catalyst such as glucose oxidase to a platinum surface of an electrode include glutaraldehyde, cyanogen bromide, hydrazine, benzoquinone, periodate, trichloro-s-triazine, tosyl chloride and diazonium compounds. Each of these crosslinking agents is suitable for immobilization of proteins such as glucose oxidase by coupling to a primary amino functional silane coupling agent, with the exception of the diazonium which is couplable to a phenol or aromatic amine functional silane coupling agent. Further, the trichloro-s-triazine cross-linking agent can crosslink the enzyme through a hydroxyl functional group of a silane coupling agent and tosyl chloride is couplable to a thiol functional group of a silane coupling agent. Some of the suitable silane coupling agents include 3-amino-propyltriethoxysilane; N-2-aminoethyl-3-aminopropyltrimethoxysilane; 4-aminobutyldimethylmethoxysilane; (aminoethylaminomethyl)phemethyltrimethoxysilane; 4-aminobutyltriethoxysilane; N-(2-aminoethyl)-3-aminomethyldimethoxysilane; and 3-aminopropyltris(trimethylsiloxy)silane and the like.

Once the catalyst 114 has been bonded to the anode 122, the membrane 112 is applied over the enzyme 114 and working surface 127. The membrane materials described herein are very compatible with whole blood, have a durable surface and are highly selective to oxygen penetration so that a sufficient stoichiometric excess of oxygen permeates the membrane 112 even from whole blood.

A surprising characteristic of the polymerized silicon-containing membrane 112 is glucose-permeability which is contrary to the teaching of the prior art. The prior art teaches placement of a silicone rubber secondary membrane 16 between the enzyme layer 14 and the electrode 18 to kept interferants, including glucose, away from the electrode 18. In the prior art, therefore, the glucose reaction occurs away from the electrode 18 reducing the efficiency of sensor 10.

Another surprising characteristic of the membrane 112 is its ability to prevent passage of abscorbic acid to electrode 118. Absorbic acid is a major interferant and is essentially prevented from reaching the electrode surface 118 by membrane 112 whereas in the prior art, this interferant passed through the primary membrane 12 and secondary membrane 16 to a much greater extent. Further, the cured membrane 112 has a durable and resilient surface allowing the membrane 112 to be rinsed and wiped off after use to remove any contaminents that could build up and foul the membrane.

The preferred materials for membrane 112 are an anionically stabilized, water-based hydroxyl endblocked polydimethylsiloxane elastomer containing about 5 percent by weight colloidal silica sold by Dow Corning as elastomer and manufactured in accordance with Dow Corning U.S. Pat. No. 4,221,668. To show the new and unexpected results using membranes 112 formed from a dispersion of polymerizable silicon-containing compounds applied in layer form in an incompletely cured state dispersed in a removable liquid carrier, four membranes were made-three from silicone-water liquid dispersions and one from a silicone paste material having essentially no removable liquid phase. The membranes were prepared by casting the elastomers onto a polyester film with a 0.25 mm (10 mil.) doctor blade and curing at ambient conditions. The three compositions having a removable carrier (water) were applied as a neat polysiloxane emulsions.

Curing was accomplished in 30-60 minutes, but can be accelerated with heat. This process gave a final dry film (membrane) thickness of approximately 0.11 mm (4.5 mils.).

The three carrier-removable silicone latex compositions from Dow Corning differ only slightly in material composition. Dow Corning 3-5024 is the base system containing hydroxyl endblocked dimethylpolysiloxane elastomer with 5 percent by weight $SiO_2$, and an anionic emulsifier and may also include a suitable cross-linking agent such as a silane and catalyst, such as an alkyl tin salt. This material is the least viscous (1000 cps) of the three and cures to a thin clear film.

A second silicone water based elastomer, Dow Corning 3-5025, identical to Dow Corning 3-5024 with the addition of an organic, thixotropic additive, has a precured viscosity of 25000 cps. This film is also clear on drying.

A third silicone water based elatomer, Dow Corning 3-5035, includes about 4.5 percent by weight $TiO_2$ filler. These films are opaque and white in color.

A heat-curable silicone paste (Dow Corning 3-9595) having essentially no volatizable carrier was also tested for comparison purposes. Dow Corning 3-9595 is a dimethylpolysiloxane elastomer containing 40 percent by weight silica and is supplied as a two-part putty-like material requiring the material to be spread into a layer using a doctor blade.

Results of the evaluation of membranes made from the above-identified four materials are summarized in the following table:

FLUX DENSITY (J) AND PERMEABILITY (P) OF SILICONE RUBBERS

| MATERIAL | GLUCOSE | URIC ACID | ASCORBIC ACID | $H_2O_2$ |
|---|---|---|---|---|
| DC3-5024 Clear Latex | J=1.78 X $10^{-11}$ | 7.45 X $10^{-14}$ | 5.79 X $10^{-14}$ | 1.60 X $10^{-12}$ |
| | P=7.31 X $10^{-9}$ | 2.12 X $10^{-10}$ | 2.04 X $10^{-10}$ | 6.88 X $10^{-9}$ |
| DC3-5025 Clear/ Latex | J=2.97 X $10^{-11}$ | 1.2 X $10^{-12}$ | 4.6 X $10^{-15}$ | 9.12 X $10^{-13}$ |
| | P=1.22 X $10^{-8}$ | 3.5 X $10^{-9}$ | 1.6 X $10^{-11}$ | 3.9 X $10^{-9}$ |
| DC3-5035 White/ Latex | J=8.49 X $10^{-11}$ | 3.6 X $10^{-12}$ | 6.9 x $10^{-14}$ | 2.3 X $10^{-11}$ |
| | P=3.49 X $10^{-8}$ | 9.8 X $10^{-9}$ | 2.5 X $10^{-10}$ | 9.9 X $10^{-8}$ |
| DC3-9595 putty consistency | J=7.41 X $10^{-13}$ | 1.5 X $10x^{-14}$ | 3.8 X $10^{-14}$ | 1.9 X $10^{-13}$ |
| | P=3.05 X $10^{-11}$ | 4.2 X $10^{-11}$ | 1.4 X $10^{-10}$ | 8.5 X $10^{-10}$ |

J = flux density (moles $cm^{-2}$ $sec^{-1}$)

P = permeability coefficient ($cm^2$ $sec^{-1}$)

Quite surprisingly, the glucose permeability of the silicone material in paste form having essentially no volatizable is three orders of magnitude lower than Dow Corning 3-5025 and Dow Corning 3-5035 and two orders of magnitude lower than Dow Corning 3-5024. The evaluation table also emphasizes the three latex materials are much more selective for glucose relative to ascorbic acid than the paste form silicone.

Sensor 100 was tested and the relationship between glucose concentration in mg/dl and sensor current in nanoamperes is plotted in FIG. 5. One of the significant features of sensor 100 as graphically illustrated in

FIG. 5 is the linear relationship of glucose concentration to sensor current. Membrane 112 is both glucose and oxygen-permeable and a stoichiometric excess of oxygen to glucose permeates the membrane 112 from whole blood which results in the linear relationship from the low end 125 to the high end 128 of the graph (FIG. 5). If the oxygen permeability of membrane 112 were lower, an insufficient supply of oxygen would be provided and the linear relationship would be lost. Membrane 112, however, allows passage of sufficient oxygen to provide a high dynamic reading by sensor 100 from 0 to 600 and above using whole blood (FIG. 5) whereas prior art sensors 10 are only able to attain an upper limit of 500 or less using diluted blood.

Having described the components of sensor 100, the preparation of electrode 118 is now described. Preparation of electrode 118 consists of two steps: 1) enzyme (catalyst) immobilization and 2) formation of membrane 112. The first step of catalyst immobilization involves preparation of an activated platinum anode surface capable of bonding to the catalyst 114, e.g., through a suitable crosslinking agent. This is accomplished, for example, by coating the cleaned anode 122 with, for example, 0.5 uL of 100% aminofunctional silane coupling agent, such as N-beta-(aminoethyl)gamma-aminopropyltrimethoxy silane and spreading the drops with a wire over the entire outer surface of the anode 122. The electrode 118 then is allowed to stand for at least two minutes after which the surface is washed with water or acetone to remove excess silane. Only the platinum anode surface 122 is treated; the epoxy 124 and silver cathode 126 surfaces are not intentionally treated, although application thereto does not interfere with electrode performance.

A cross-linking agent, preferably glutaraldehyde (25% by weight in water) then is applied to the amino activated platinum anode surface 122 to permit subsequent coupling of the catalyst, e.g., glucose oxidase or glucose dehydrogenase 114. About 0.5 uL of the glutaraldehyde is applied to the derivatized platinum surface such as by applying the solution dropwise with a wire and manually spreading the drops over the anode surface using the wire. Miles HPL 500 glucose oxidase (0.5 uL of 5000 IU/mL) is applied in the same manner to the pretreated platinum anode 122. The sensor 100 is allowed to dry and is rinsed with water several times to remove any unreacted glutaraldehyde and enzyme catalyst. The electrode 118 is tested for successful enzyme coupling by applying an aqueous glucose solution. Within ten seconds the electrode 100 should give a current response of greater than 500 nA for a 500 mg/dL glucose solution.

The entire working surface 127 of electrode 118 is then treated with the silane coupling agent and dried. Dow Corning 3-5035 silicone latex (40% by weight solids) is diluted with an equal volume of distilled water and about two drops are spread evenly over the entire working surface 127 of electrode 118. The latex cures upon drying to form a bonded membrane 112 on the working surface 127.

Sensor 100 with the single silicone water based elastomer membrane 112 and the placement of the enzyme 114 directly on the anode 122 of electrode 118 is the first home use glucose testing apparatus capable of using whole blood. Sensor 100 has a short response time of one minute and is reusable. There is no user bias and sensor 100 is capable of unlimited tests per month. Due to the durability of membrane 112 and the inexpensive components, sensor 100 is cost competitive with the existing form of home testing, the reagent strip, while demonstrating an accuracy within 2-3%.

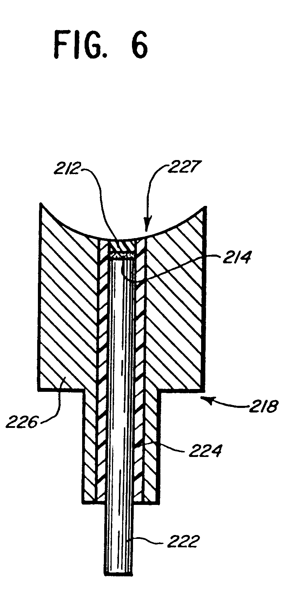

In the present invention it has been determined that only the anode area of the working surface of the electrode needs to be covered by the membrane of the present invention. In an alternative embodiment of the invention (FIG. 6), only the portion of the anode 222 in the working surface 227 is covered by the membrane 212. The anode 222 is an independent element of electrode 218 with the portion forming the working surface 227 including the enzyme layer 214 and membrane 212. The anode 222 in electrode 218 is the sole part requiring replacement and may be constructed to be removed and replaced with a new anode 222 thereby saving the cost of replacing the entire electrode 218.

Although sensor 100 has been described as including an electrode 118, it is to be understood the unique features of the present system could also apply to transistor readout systems. In a transistor readout system enzyme 114 would be applied to a selected junction of the transistor and membrane 112 would be applied over the enzyme 114 and junction. Previously, transistors could not be used since they are very sensitive to contaminants. The membranes 112 of the present invention, however, insulate the transistor from contaminants while allowing the passage of glucose and oxygen.

Sensor 100 can also be used to measure glucose in other body fluids such as sweat. To perform measurements of this type, sensor 100 is placed in tight contact with the skin. The glucose permeability of the membrane 112 is tailored to be less than that of the skin by selection of the proper membrane material. The sensor 100 response will then be proportional to the blood glucose. A preferred form would be a wrist watch type sensor with a digital display of the glucose concentration. Conceptually other advances in technology could also be incorporated such as alarms for high and low glucose or an alarm to remind the wearer that the time for insulin injection has come.

Many modifications and variations of the present invention are possible in light of the above teachings. Thus, it is to be understood that, within the scope of the appended claims, the invention may be practiced other than as specifically described.

## Claims

1. An electrochemical sensor (100) for determining the relative concentration of glucose in a blood sample by using an enzyme catalyzed reaction, said sensor comprising an anode (122) and a cathode (126) which are electrically insulated from each other, a glucose permeable membrane (112) for protecting the electrodes (122, 126) from contaminants and the enzyme being disposed between the membrane (112) and the anode (122), the enzyme consisting of glucose oxidase (114), wherein the glucose oxidase is immobilized on an outer surface of said anode (122) and the glucose permeable membrane (112) is characterized as an oxygen permeable, hydroxyl endblocked diorganopolysiloxane, having an average molecular weight of at least 5,000, the membrane (112) covering the glucose oxidase immobilized anode (122), thereby retaining said glucose oxidase (114) between said membrane (112) and said anode (122).

2. The electrochemical sensor of Claim 1, wherein the anode (122) is platinum and the cathode (126) is silver.

3. The electrochemical sensor of Claim 1, wherein the anode (122) and cathode (126) are electrically insulated from each other by epoxy (124).

4. The electrochemical sensor of Claim 1, in which the membrane (112) is an oxygen permeable, glucose permeable, hydroxyl endblocked dimethylpolysiloxane, having an average molecular weight of at least 5,000.

## Patentansprüche

1. Elektrochemischer Sensor (100) zur Bestimmung der relativen Glucosekonzentration in einer Blutprobe unter Verwendung einer Enzym-katalysierten Reaktion, wobei der Sensor eine Anode (122) und eine Kathode (126), welche voneinander elektrisch isoliert sind, und eine Glucose-permeable Membran (112) zum Schutz der Elektroden (122, 126) vor Kontaminanten umfaßt und das Enzym zwischen der Membran (112) und der Anode (122) angeordnet ist, wobei das Enzym aus Glucoseoxidase (114) besteht und die Glucoseoxidase auf einer äußeren Oberfläche der Anode (122) immobilisiert ist, dadurch gekennzeichnet, daß die Glucose-permeable Membran (112) eine Sauerstoff-permeable, Hydroxyl-endblockierte Diorganopolysiloxanmembran mit einem durchschnittlichen Molekulargewicht von mindestens 5000 ist, wobei die Membran (112) die Glucoseoxidase-immobilisierte Anode (122) bedeckt, wodurch die Glucoseoxidase (114) zwischen der Membran (112) und der Anode (122) fixiert wird.

2. Elektrochemischer Sensor nach Anspruch 1, dadurch gekennzeichnet, daß die Anode (122) aus Platin und die Kathode (126) aus Silber besteht.

3. Elektrochemischer Sensor nach Anspruch 1, dadurch gekennzeichnet, daß die Anode (122) und die Kathode (126) voneinander elektrisch durch Epoxy (124) isoliert sind.

4. Elektrochemischer Sensor nach Anspruch 1, dadurch gekennzeichnet, daß die Membran aus (112) einem Sauerstoff-permeablen, Glucose-permeablen, Hydroxyl-endblockierten Dimethylpolysiloxan mit einem durchschnittlichen Molekulargewicht von mindestens 5000 besteht.

## Revendications

1. Détecteur électrochimique (100) pour la détermination de la concentration relative du glucose dans un échantillon de sang au moyen d'une réaction catalysée par un enzyme, ledit détecteur comprenant une anode (122) et une cathode (126) qui sont isolées électriquement l'une de l'autre, une membrane (112) perméable au glucose pour protéger les électrodes (122, 126) des impuretés, et l'enzyme placé entre la membrane (112) et l'anode (122), l'enzyme consistant en glucose-oxydase (114), dans lequel la

glucose-oxydase est immobilisée sur une surface extérieure de ladite anode (122) et la membrane (112) perméable au glucose possède pour caractéristique de consister en un diorganopolysiloxane à blocs terminaux hydroxyle ayant un poids moléculaire moyen d'au moins 5000, la membrane (112) recouvrant l'anode (122) portant la glucose-oxydase immobilisée, ce qui retient ladite glucose-oxydase (114) entre ladite membrane (112) et ladite anode (122).

**2.** Détecteur électrochimique suivant la revendication 1, dans lequel l'anode (122) est constituée de platine et la cathode (126) est constituée d'argent.

**3.** Détecteur électrochimique suivant la revendication 1, dans lequel l'anode (122) et la cathode (126) sont isolées électriquement l'une de l'autre par une résine époxy (124).

**4.** Détecteur électrochimique suivant la revendication 1, dans lequel la membrane (112) est un diméthylpolysiloxane à blocs terminaux hydroxyle perméable à l'oxygène et perméable au glucose, ayant un poids moléculaire moyen d'au moins 5000.

FIG. 1 PRIOR ART

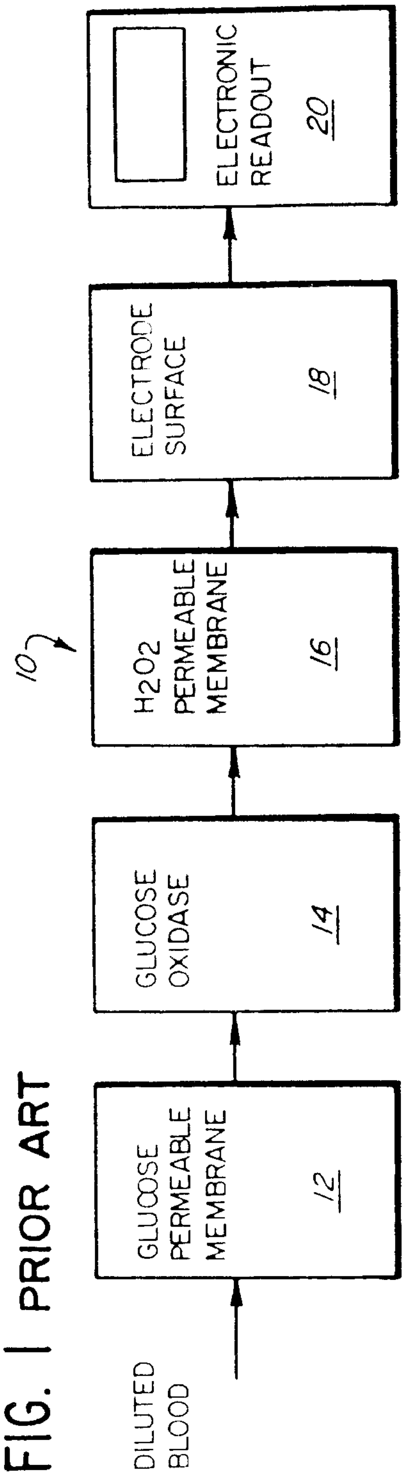


FIG. 2

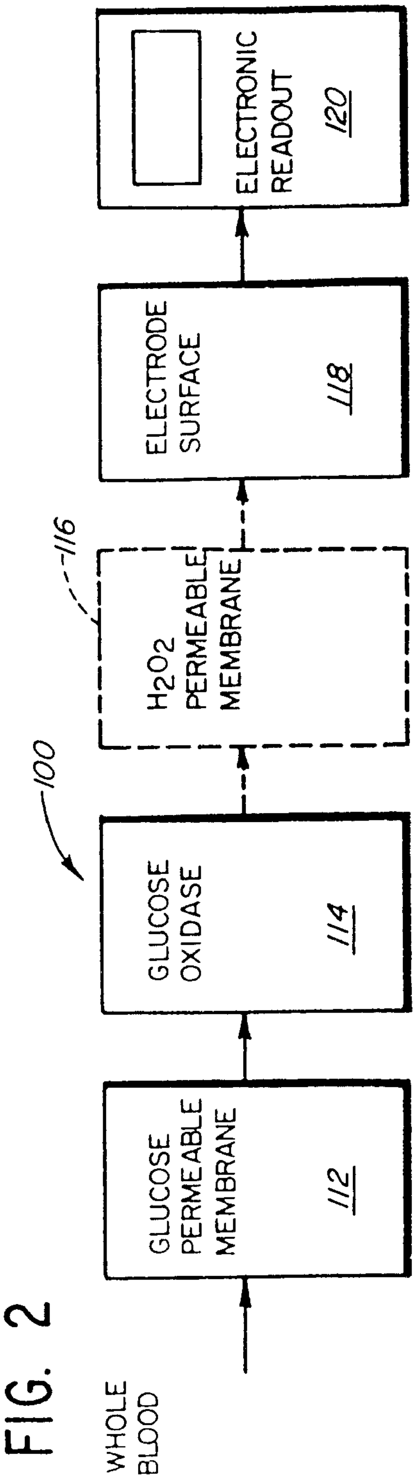

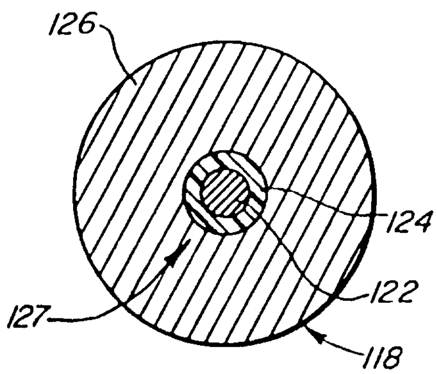


FIG. 4

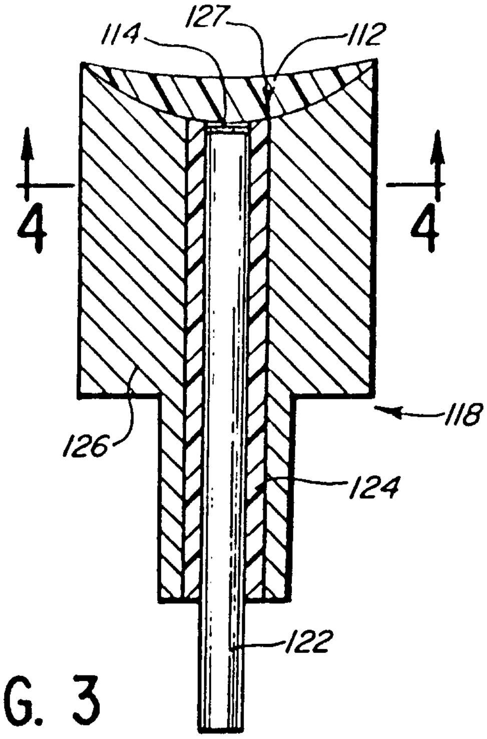


FIG. 3

FIG. 5

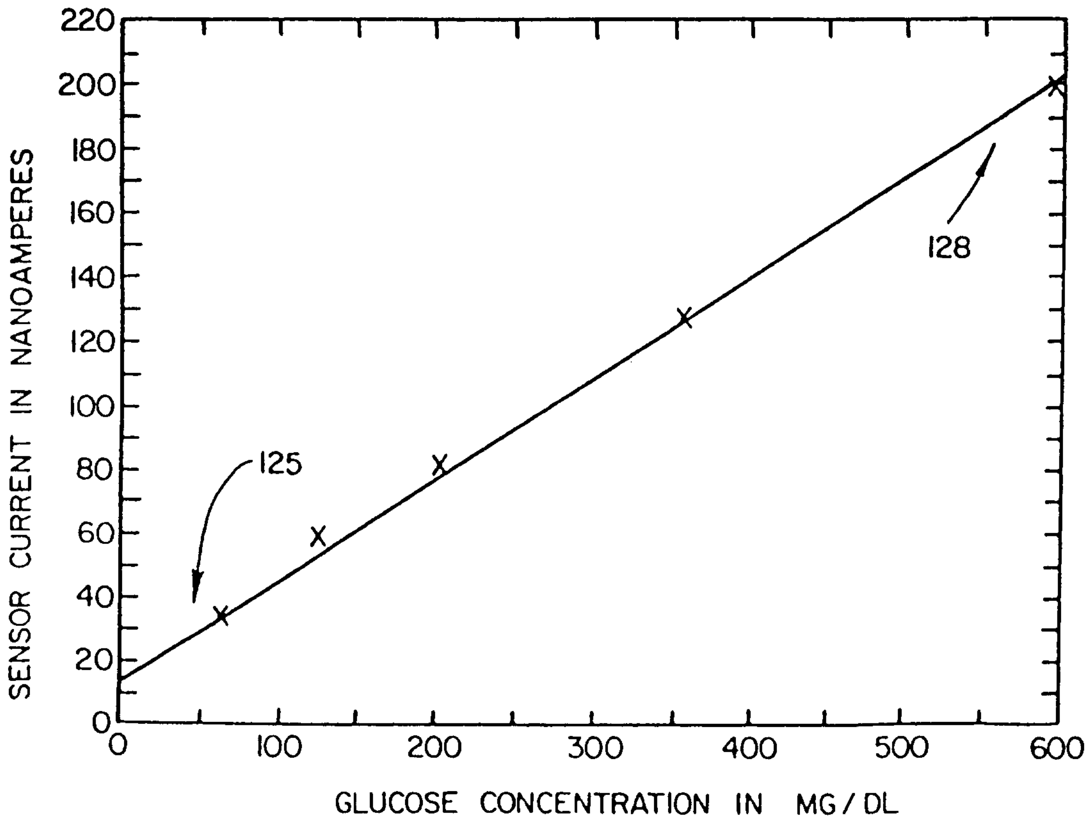

FIG. 6